(19) **Europäisches Patentamt**
**European Patent Office**
**Office européen des brevets**

(11) **EP 4 559 392 A1**

(12) **EUROPEAN PATENT APPLICATION**
published in accordance with Art. 153(4) EPC

(43) Date of publication:
**28.05.2025 Bulletin 2025/22**

(21) Application number: **23842895.7**

(22) Date of filing: **12.07.2023**

(51) International Patent Classification (IPC):
**A61B 5/1455** (2006.01)      **A61B 5/02** (2006.01)
**A61B 5/08** (2006.01)

(52) Cooperative Patent Classification (CPC):
**A61B 5/00; A61B 5/02; A61B 5/08; A61B 5/1455**

(86) International application number:
**PCT/JP2023/025792**

(87) International publication number:
**WO 2024/018972 (25.01.2024 Gazette 2024/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.07.2022   JP 2022115117**

(71) Applicant: **OMRON Corporation**
**Kyoto 600-8530 (JP)**

(72) Inventors:
 • **YAMAMOTO, Kazuo**
   **Kyoto-shi, Kyoto 600-8530 (JP)**

 • **OKUNO, Yutaro**
   **Kyoto-shi, Kyoto 600-8530 (JP)**
 • **MATSUI, Yuki**
   **Kyoto-shi, Kyoto 600-8530 (JP)**
 • **KIGUCHI, Tetsuya**
   **Kyoto-shi, Kyoto 600-8530 (JP)**
 • **ARAKAWA, Masayuki**
   **Kyoto-shi, Kyoto 600-8530 (JP)**
 • **OHASHI, Suguru**
   **Kyoto-shi, Kyoto 600-8530 (JP)**
 • **KUBO, Mitsuaki**
   **Kyoto-shi, Kyoto 600-8530 (JP)**

(74) Representative: **SONN Patentanwälte GmbH & Co KG**
**Riemergasse 14**
**1010 Wien (AT)**

(54) **OXYGEN SATURATION DEGREE MEASUREMENT DEVICE AND OXYGEN SATURATION DEGREE MEASUREMENT METHOD**

(57)      An oxygen saturation measuring device has: a band wound on a wrist of a measurement subject; a first sensor portion attached to the band, the first sensor portion acquiring pulse wave signals of an artery of the wrist, and measuring oxygen saturation of the artery of the wrist on the basis of the acquired pulse wave signals; and a notification portion that, in a case in which the oxygen saturation measured by the first sensor portion satisfies a preset condition, provides the measurement subject with a warning expressing a possibility of respiratory organ disorder.

FIG.1

EP 4 559 392 A1

## Description

Technical Field

The present disclosure relates to an oxygen saturation measuring device and an oxygen saturation measuring method.

Background Art

A touchscreen interface equipped with a pulse oximeter such as in Japanese Patent Application Laid-Open (JP-A) No. 2010-246894 has conventionally been disclosed as a device that measures oxygen saturation ($SpO_2$) in arterial blood. In JP-A No. 2010-246894, the oxygen saturation in the arterial blood of a fingertip of a measurement subject can be measured by the fingertip being made to contact the touchscreen.

Patent Document 1: JP-A No. 2010-246894

SUMMARY OF INVENTION

Technical Problem

Generally, when oxygen saturation falls outside of a predetermined range, it can be assumed that the state of the respiratory organs of the measurement subject is disorderly . For example, in a case in which the oxygen saturation is within the preset range of around 95% to around 99%, it can be judged that the state of the respiratory organs of the measurement subject is normal. On the other hand, in a case in which the oxygen saturation falls outside of the set range such as is 94% for example, it can be judged that the state of the respiratory organs of the measurement subject is disorderly.

Here, if the oxygen saturation of a measurement subject can be measured continuously over time, temporal changes in the measured value can be obtained. By using temporal changes in the measured value, it becomes possible to more generally diagnose the state of the respiratory organs of the measurement subject, and as a result, an improvement in the state of the respiratory organs can be devised. In order to continuously measure oxygen saturation over time, the oxygen saturation measuring device must be continuously over time placed on a predetermined position of the body of the measurement subject.

However, in a case in which the contacting portion of the measurement subject that is used at the time of measuring oxygen saturation is the fingertip as in JP-A No. 2010-246894, the oxygen saturation measuring device is continuously worn on a fingertip. Therefore, for example, actions of work such as holding a pen or operating an electronic terminal by the fingertips, and actions of daily life such as eating or excretion, are easily impeded. Namely, it is difficult to continuously measure the oxygen saturation over time in a case in which the oxygen saturation is measured at the position of a fingertip.

The present disclosure was made while focusing on the above-described circumstances, and provides an oxygen saturation measuring device and an oxygen saturation measuring method by which a measurement subject can easily measure oxygen saturation continuously over time, and that can devise improvement in the state of respiratory organs. Solution to Problem

An oxygen saturation measuring device relating to a first aspect of the present disclosure has: a band wound on a wrist of a measurement subject; a first sensor portion attached to the band, the first sensor portion acquiring pulse wave signals of an artery of the wrist, and measuring oxygen saturation of the artery of the wrist on the basis of the acquired pulse wave signals; and a notification portion that, in a case in which the oxygen saturation measured by the first sensor portion satisfies a preset condition, provides the measurement subject with a warning expressing a possibility of respiratory organ disorder.

In accordance with the first aspect, the band is wound on the wrist of the measurement subject, and pulse wave signals of an artery of the wrist are acquired by the first sensor portion that is attached to the band. Further, the oxygen saturation of the artery of the wrist is measured on the basis of the acquired pulse wave signals. Namely, because the oxygen saturation is measured at the position of the wrist, as compared with a case of measuring the oxygen saturation at the position of a fingertip, it is difficult for a state in which actions of work or actions of daily life of the measurement subject are impeded to arise, and it is easy to continuously measure the oxygen saturation over time.

Further, in a case in which the measured oxygen saturation of the artery of the wrist satisfies a preset condition, a warning expressing the possibility of the respiratory organ disorder is provided to the measurement subject. Due to the warning, the measurement subject is urged to take action such as conferring with a doctor for example. Therefore, as a result, an improvement in the state of the respiratory organs can be devised.

In an oxygen saturation measuring device relating to a second aspect, in the first aspect, the first sensor portion is attached to an inner side of the band.

In accordance with the second aspect, because the first sensor portion is attached to the inner side of the band, i.e., the side contacting the wrist, it is easy to measure, for example, the radial artery that is near to the surface of the inner side of the wrist.

In an oxygen saturation measuring device relating to a third aspect, in the first aspect or the second aspect, in a case in which the measured oxygen saturation does not reach a preset reference value, the notification portion provides, as the warning, a notice urging the measurement subject to measure oxygen saturation at a position of a fingertip.

In accordance with the third aspect, in a case in which the measured oxygen saturation does not reach a preset reference value, a notice urging the measurement subject to measure oxygen saturation at the position of a fingertip is given as a warning to the measurement subject. Therefore, measurement of the oxygen saturation with even higher measurement accuracy can be promoted.

In an oxygen saturation measuring device relating to a fourth aspect, any of the first aspect through the third aspect further has a second sensor portion attached to the band and measuring oxygen saturation of an artery of a fingertip of the measurement subject.

In accordance with the fourth aspect, the second sensor portion, which is attached to the band and measures the oxygen saturation of an artery of a fingertip, is further provided. Therefore, both continuous measurement over time of oxygen saturation at the position of the wrist, and measurement of oxygen saturation at the position of a fingertip with higher measuring accuracy, can be realized by the one oxygen saturation measuring device. Namely, there is no need to separately ready an oxygen saturation measuring device for measuring the oxygen saturation of an artery of a fingertip.

In an oxygen saturation measuring device relating to a fifth aspect, in the fourth aspect, the second sensor portion is attached to an outer side of the band.

In accordance with the fifth aspect, the second sensor portion is attached to the outer side of the band, i.e., the side of the band that does not contact the wrist, at a position that can be visually recognized easily by the measurement subject. Therefore, as compared with a case in which the second sensor portion is attached to the inner side of the band, handling of the second sensor portion by the measurement subject is easy.

An oxygen saturation measuring device relating to a sixth aspect of the present disclosure has: a band wound on a wrist of a measurement subject; a first sensor portion attached to the band, the first sensor portion acquiring pulse wave signals of an artery of the wrist, and measuring oxygen saturation of the artery of the wrist on the basis of the acquired pulse wave signals; a notification portion that, in a case in which the oxygen saturation measured by the first sensor portion satisfies a preset condition, provides the measurement subject with a warning expressing a possibility of respiratory organ disorder; a second sensor portion attached to the band and measuring oxygen saturation of an artery of a fingertip of the measurement subject; and a display portion displaying the oxygen saturation measured by the second sensor portion.

In accordance with the sixth aspect, both continuous measurement over time of oxygen saturation at the position of the wrist, and measurement of oxygen saturation at the position of a fingertip with higher measuring accuracy, can be realized by using the one oxygen saturation measuring device.

In an oxygen saturation measuring device relating to a seventh aspect, in the sixth aspect, the notification portion and the display portion form an integrated body.

In accordance with the above-described structure, the overall dimensions of the oxygen saturation measuring device can be reduced as compared with a case in which the notification portion and the display portion are separate bodies. Therefore, the oxygen saturation measuring device can be structured to be compact.

An oxygen saturation measuring method relating to an eighth aspect of the present disclosure includes: acquiring pulse wave signals of an artery of a wrist of a measurement subject; measuring oxygen saturation of the artery on the basis of the acquired pulse wave signals; and, in a case in which the measured oxygen saturation satisfies a preset condition, providing the measurement subject with a warning.

In accordance with the eighth aspect, in the same way as in the first aspect, it is easy to continuously measure the oxygen saturation over time, and an improvement in the state of respiratory organs can be devised.

Advantageous Effects of Invention

In accordance with the oxygen saturation measuring device and oxygen saturation measuring method relating to the present disclosure, it is easy for a measurement subject to continuously measure their oxygen saturation over time, and an improvement in the state of respiratory organs can be devised.

BRIEF DESCRIPTION OF DRAWINGS

Fig. 1 is a perspective view explaining an oxygen saturation measuring device relating to an embodiment of the present disclosure.

Fig. 2 is a cross-sectional view explaining the oxygen saturation measuring device relating to the present embodiment.

Fig. 3 is a plan view explaining a sensor unit of the oxygen saturation measuring device relating to the present embodiment.

Fig. 4 is a cross-sectional view along line 4-4 of Fig. 3.

Fig. 5 is a graph explaining changes in values, which are measured continuously over time, of oxygen saturation of an artery of a fingertip and changes in values, which are measured continuously over time, of oxygen saturation of an artery of a wrist.

Fig. 6 is a flowchart explaining an oxygen saturation measuring method using the oxygen saturation measuring device relating to the present embodiment.

Fig. 7 is a perspective view explaining a state of measuring oxygen saturation of an artery of a fingertip by using a second sensor portion, in the oxygen saturation measuring method relating to the present embodiment.

Fig. 8 is a flowchart explaining a state of measuring oxygen saturation of an artery of a fingertip by using the second sensor portion, in an oxygen saturation measuring method relating to a modified example.

DESCRIPTION OF EMBODIMENTS

A present embodiment is described hereinafter. Same portions or similar portions are denoted by same or similar reference numerals in the following description of the drawings. However, the drawings are schematic, and the relationships between the thicknesses and the planar dimensions, and the ratios of the thicknesses of respective devices and respective members, and the like differ from those in actuality. Accordingly, specific thicknesses and dimensions are to be judged by taking the following description into consideration. Further, the respective drawings as well include portions at which the relationships and ratios of dimensions differ from one another. Moreover, the numbers of the respective structural elements of the present disclosure are not limited to one, and pluralities thereof may exist, unless otherwise specified in the specification.

<Oxygen Saturation Measuring Device>

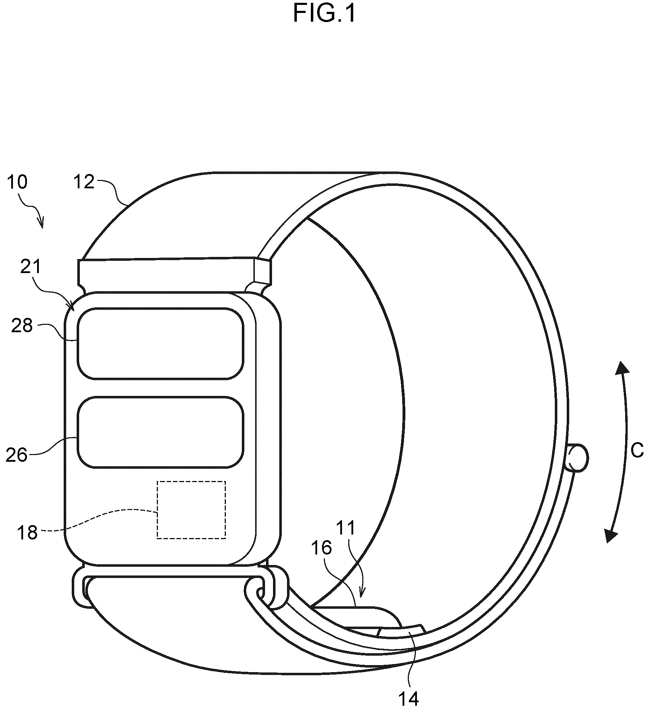

The structure of an oxygen saturation measuring device 10 relating to a present embodiment is described with reference to Fig. 1 through Fig. 5. As illustrated in Fig. 1, the oxygen saturation measuring device 10 relating to the present embodiment has a band 12, a first sensor portion 11, a notification portion 26 and a second sensor portion 21.

(Band)

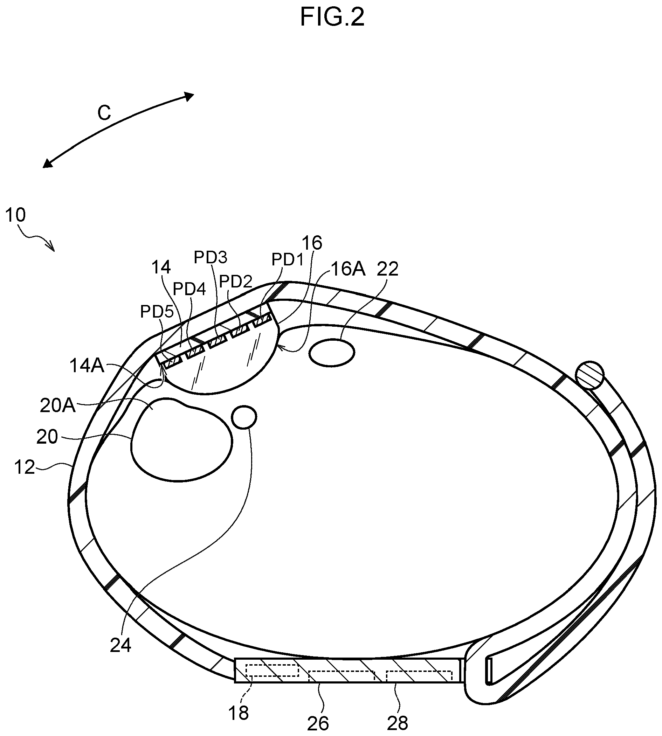

As illustrated in Fig. 2, the band 12 is wound on the wrist of a measurement subject. The material of the band 12 is an arbitrary material such as resin, fabric or metal. Further, a fastener for adjusting and fixing the length when the band 12 is wound around the wrist is provided at the band 12.

(First Sensor Portion)

As illustrated in Fig. 1 and Fig. 2, the first sensor portion 11 is attached to the inner side of the band 12, i.e., the side of the band 12 that contacts the wrist. Note that, in the present disclosure, the mounted position of the first sensor portion is not limited to the inner side of the band 12, and may be the outer side of the band 12. The first sensor portion 11 acquires pulse wave signals of an artery of the wrist, and measures the oxygen saturation of the artery of the wrist on the basis of the acquired pulse wave signals.

Specifically, the first sensor portion 11 of the present embodiment is structured by a base portion 14, contacting portions 16, light-emitting elements, light-receiving elements, and a computing section 18. The computing section 18 is housed within a case 25. Note that, in the present disclosure, the first sensor portion 11 can be structured arbitrarily provided that it can acquire the pulse wave signals of an artery of the wrist.

In the oxygen saturation measuring device 10 exemplified in Fig. 2, first light-receiving element PD1, second light-receiving element PD2, third light-receiving element PD3, fourth light-receiving element PD4 and fifth light-receiving element PD5 are provided as the light-receiving elements. Further, radius 20, radial styloid process 20A, flexor carpi radialis tendon 22 and radial artery 24 are exemplified at the interior of the wrist in Fig. 2.

(Base Portion)

The base portion 14 is provided on the inner surface of the band 12. Specifically, in the present embodiment, the base portion 14 can be fixed to the band 12 by adhesion for example. However, the method of fixing is not limited to adhesion, and a plate-shaped member for fixing may be interposed between the base portion 14 and the band 12, and a circuit board may be fixed to the plate-shaped member by fastening screws or the like.

The base portion 14 has, between the band 12 and the contacting portions 16, a joining surface 14A that is flat. In the present embodiment, both the light-emitting elements and the light-receiving elements are joined to the joining surface 14A of the base portion 14. In the present disclosure, it suffices for at least either one of the light-emitting elements and the light-receiving elements to be joined to the joining surface 14A.

Further, in the present disclosure, the base portion 14 may itself be a circuit board on which the light-emitting elements and the light-receiving elements are mounted, or may be a combination of a circuit board and a plate-shaped member for fixing, or may be a combination of a circuit board and a shock-absorbing member. Namely, it suffices for the base portion 14 of the present disclosure to have the joining surface 14A to which at least one of the light-emitting elements and the light-receiving elements are joined.

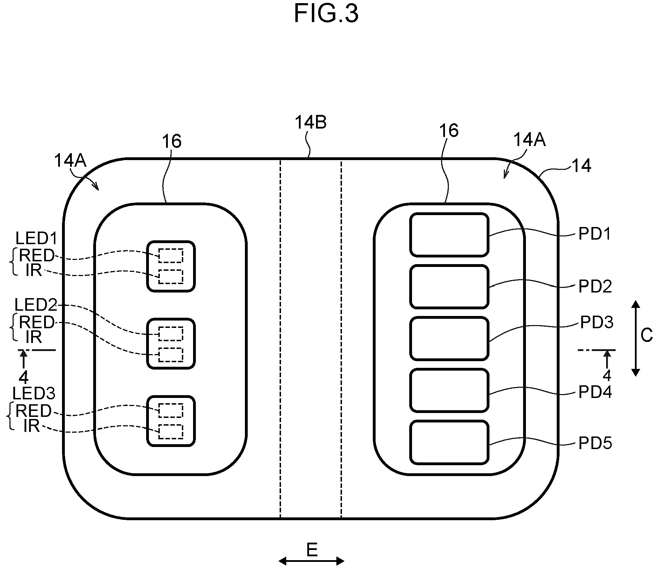

As illustrated in Fig. 3, the light-emitting elements and the light-receiving elements are disposed on the joining surface 14A of the base portion 14. Note that illustration of the circuit board to which the light-emitting elements and the light-receiving elements are mounted is omitted.

(Light-Emitting Elements)

The light-emitting elements are electronic parts such as light-emitting diodes (LEDs) for example. The light-emitting elements are disposed at preset positions with respect to the contacting portion 16, and irradiate light toward an artery of the wrist. In the present embodiment, a first light-emitting element LED1, a second light-emitting element LED2 and a third light-emitting element LED3 are provided as the light-emitting elements. The first light-emitting element LED1, the second light-emitting element LED2 and the third light-emitting element LED3 are disposed so as to be apart from one another, and are lined-up along the peripheral direction of the wrist. Note that, in the present disclosure, the number of light-emitting elements is an arbitrary number of one or more.

As illustrated in Fig. 3, the first light-emitting element LED1, the second light-emitting element LED2 and the third light-emitting element LED3 each have a group of light sources that includes one or more red light sources RED and one or more infrared light sources IR. Namely, in the present embodiment, two types of light are irradiated from one light-emitting element. Further, the two types of reflected light that the one light-receiving element receives are used in measuring the oxygen saturation. Note that, in the present disclosure, the number of types of light sources is not limited to two types, and may be one type or may be three or more types.

(Principles of Oxygen Saturation Measurement)

Here, the principles of oxygen saturation measurement in the present embodiment in which two types of irradiated light and two types of reflected light are used, are described. Specifically, for example, red light of a wavelength region of around 620 nm to around 700 nm has the characteristic that the light absorption degree thereof varies greatly depending on the absence/presence of oxygen that binds to hemoglobin. On the other hand, infrared light of a wavelength region of around 850 nm to around 960 nm has the characteristic that the light absorption degree thereof does not vary greatly depending on the absence/presence of oxygen that binds to hemoglobin.

Therefore, changes in the intensity of the pulse wave signal of the reflected light of the red light are monitored over time, and the fluctuating component (in other words, the AC) of the pulse wave signal that is included in the monitored pulse wave signal, and the fixed component that does not fluctuate (in other words, the DC), are computed. Then, by dividing the fluctuating component by the fixed component (AC/DC), the perfusion index (PI) value of the red light is computed as $PI_{RED}$. Further, in the same way as the red light, by monitoring the changes in the intensity of the pulse wave signal of the reflected light of the infrared light over time, the PI value ($PI_{IR}$) of the reflected light of the infrared light is computed.

Then, the ratio ($PI_{RED}/PI_{IR}$) of the PI value ($PI_{RED}$) of the red light and the PI value ($PI_{IR}$) of the infrared light is computed. Then, the oxygen saturation can be computed by using the computed ratio ($PI_{RED}/PI_{IR}$) in following formula (1).

$$\text{oxygen saturation } [\%] = a \times (PI_{RED}/PI_{IR}) + b \qquad \ldots \text{formula (1)}$$

The coefficients a, b in formula (1) can be determined by experimentation. Note that, in the present disclosure, the method of measuring the oxygen saturation is not limited to this and may be another method.

(Light-Receiving Elements)

The light-receiving elements are electronic parts such as photodiodes (PDs) for example. The light-receiving elements are disposed at preset positions with respect to the contacting portion 16, and, via contacting surface 16A of the contacting portion 16, receive light reflected from an artery of the wrist. The first light-receiving element PD1, the second light-receiving element PD2, the third light-receiving element PD3, the fourth light-receiving element PD4 and the fifth light-receiving element PD5 are disposed so as to be apart from one another, and are lined-up along the peripheral direction of the wrist. Note that, in the present disclosure, the number of light-receiving elements is an arbitrary number of one or more.

In the present disclosure, one "sensor unit" is structured by one or more light-emitting elements and one or more light-receiving elements that correspond to the one or more light-emitting elements. In the present disclosure, plural sensor units may be provided. Further, the number of light-emitting elements and the number of light-receiving elements that are included in one "sensor unit" both can be set arbitrarily.

Further, the oxygen saturation measuring device 10 relating to the present embodiment is a reflection-type device in which the intensity of pulse wave signals is measured by reflected light. However, the present disclosure is not limited to a reflection-type device, and may be a transmission-type device in which the intensity of pulse wave signals is measured by transmitted light.

(Light-Shielding Portion)

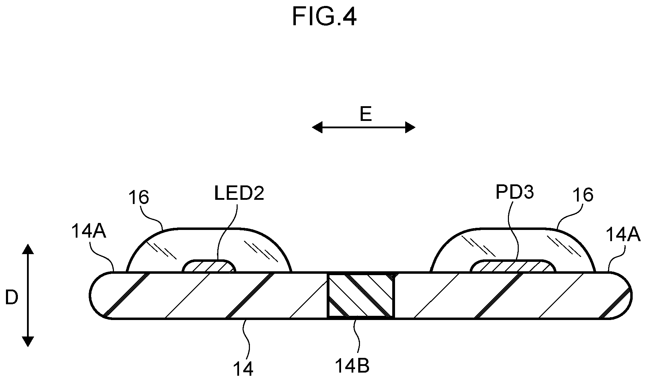

As illustrated in Fig. 4, a light-shielding portion 14B is provided between the light-emitting elements and the light-receiving elements at the base portion 14. Due to the light-shielding portion 14B, the light-receiving elements are prevented from directly receiving the light from the light-emitting elements. Note that making the interval between the light-emitting elements and the light-receiving elements as close as possible while using the light-shielding portion 14B is preferable from the standpoint of making the optical path lengths short, and as a result, improving the measurement efficiency.

(Contacting Portions)

The contacting portions 16 are attached to the band 12. The contacting portions 16 are light-transmissive with respect to the light, which is irradiated onto an artery of the wrist, and the light that is reflected from the artery of the wrist. Specifically, for example, members that are light-transmissive such as lenses can be employed as the contacting portions 16. The contacting portion 16 that is disposed at the upper side of the light-emitting elements in Fig. 4 is, for example, a diffusing lens that can enlarge the irradiated region. Further, although not illustrated, a diffusing material may be disposed at the upper side of the light-emitting elements, together with the diffusing lens or instead of the diffusing lens. Further, the contacting portion 16 that is disposed at the upper side of the light-receiving elements in Fig. 4 may be, for example, a condensing lens that can collect the reflected light.

The contacting portions 16 have the contacting surfaces 16A that protrude out from the band 12 toward the wrist side. In the present embodiment, the contacting surfaces 16A have curved shapes. The contacting surfaces 16A contact the skin of the wrist at the time of measuring oxygen saturation.

As illustrated in Fig. 3 and Fig. 4, the contacting portions 16 are dome-shaped. At the contacting surfaces 16A that correspond to the outer surfaces of the domes, due to the central portions in the left-right direction in Fig. 4 (i.e., extending direction E of the forearm) being flat, and the left and right both end portions respectively have given curvatures, the contacting surfaces 16A are curved smoothly without being angular. The curvature being 0.167 or greater for example is preferable from the standpoint of improving the fit on the measurement subject. Note that, although not illustrated, similarly, the central portions of the contacting portions 16 in wrist peripheral direction C are flat, and the both left and right end portions are respectively curved smoothly.

Further, in the present embodiment, the contacting surfaces do not have to have flat portions. The contacting surfaces may be curved shapes that are in states of having a given curvature on the whole.

Note that the "extending direction E of the forearm" in the present specification overlaps all of the direction extending along the radius 20, the direction extending along the ulna, and the direction extending along the artery. Further, strictly speaking, the "extending direction E of the forearm" differs per measurement subject. Namely, the "extending direction E of the forearm" is not determined unambiguously by coordinates in a three-dimensional space, and is determined individually on the basis of the extending direction of the radius 20, the extending direction of the ulna, and the extending direction of the artery, per measurement subject.

(Case)

The case 25 is attached to the band 12, and contacts the surface that is at the side of the back of the hand, which is the outer side of the wrist of the measurement subject. The material of the case 25 is arbitrary, such as resin or metal. Note that the case 25 is not essential in the present disclosure.

(Computing Section)

In the present embodiment, the computing section 18 is provided at the case 25. The light-receiving elements are connected to the computing section 18. Data of the pulse wave signals of the reflected lights is inputted temporally from the light-receiving elements to the computing section 18.

The computing section 18 is structured by a computer having, for example, a CPU (Central Processing Unit), a RAM (Random Access Memory), a storage device, an I/O port, and the like. The RAM, the storage device, the I/O port and the like are structured so as to be able to exchange data with the CPU via an internal bus. On the basis of the pulse wave signals acquired from the reflected lights, the computing section 18 measures the oxygen saturation of the radial artery 24, toward which light has been irradiated, by a method using the ratio of the PI values, or the like.

(Notification Portion)

As illustrated in Fig. 1, the notification portion 26 is disposed on the surface of the case 25, which surface is at the side opposite the wrist. The notification portion 26 is an image display device formed by liquid crystals for example. The notification portion 26 displays the results of computation by the computing section 18 to the exterior such that the measurement subject can visually confirm these results.

A storage device that temporarily stores the results of measurement may be provided at the notification portion 26. Note that, in the present disclosure, in a case in which the case 25 is not provided, the computing section can be disposed at a contacting portion or the base portion for example, as a portion of the computing device. Similarly, the storage device also can be provided at a member other than the case, such as at a contacting portion or the base portion. In the present disclosure, even in a case in which the notification portion 26 is not provided at the case 25, the results of computing by the computing section 18 can be obtained by, for example, using a communication section that is connected to the computing section 18 and can communicate with the exterior.

In a case in which the oxygen saturation measured by the first sensor portion 11 satisfies a preset condition, the notification portion 26 provides a warning, which expresses the possibility of respiratory organ disorder, to the measurement subject. Specifically, in a case in which the measured oxygen saturation does not reach a preset reference value, the notification portion 26 gives, as a warning, a notice urging the measurement subject to measure their oxygen saturation at the position of a fingertip.

(Reference Value)

In the present embodiment, the reference value is an oxygen saturation of 90%. If the oxygen saturation is less than 90%, the notification portion 26 gives, as a warning, a notice urging the measurement subject to measure their oxygen saturation at the position of a fingertip. Namely, the reference value in the present embodiment is a minimum value of the oxygen saturation. Further, the condition in the present embodiment is that the measured oxygen saturation has not reached the minimum value.

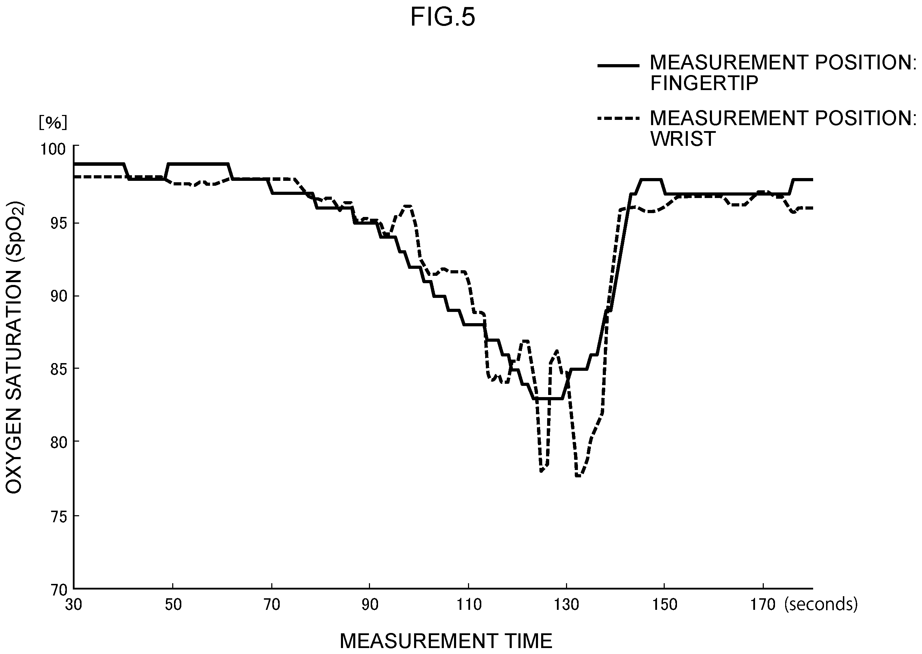

If the reference value is the oxygen saturation as in the present embodiment, the reference value that is the minimum value for judging whether or not a warning is to be given is preferably set to be within a range of 90% or more and 94% or less. The locus of the data, which is obtained by continuously measuring oxygen saturation over time by using the first sensor portion 11 at the position of a fingertip, is exemplified by the solid line in Fig. 5. Further, the locus of the data, which is obtained by continuously measuring oxygen saturation over time by using the first sensor portion 11 at the position of the wrist, is exemplified by the dashed line.

Note that, in the present disclosure, the reference value for judging whether or not a warning is to be given is not limited to the range of 90% or more and 94% or less, and can be changed in accordance with the state of the respiratory organs of the measurement subject. For example, in the case of a patient who has contracted a specific viral infection, the oxygen saturation can chronically be within the range of around 92% to around 93%. Therefore, in the present disclosure, as another example of the reference value, the reference value can be set by a combination of an initial stage value and a correction value expressing the amount of decrease from the initial stage value. The initial stage value and the correction value can respectively be set in advance per measurement subject, in accordance with the state of the respiratory organs of the measurement subject.

Specifically, for example, the average value of the oxygen saturations that have been measured within a given time period, such as the most recent one month before setting for example, can be employed as the initial stage value. For example, in a case in which the measurement subject is a patient who has contracted a specific viral infection, the initial stage value can be set to be 92% for example. However, the present disclosure is not limited to this in actuality, and the initial stage value can be changed in accordance with the measurement subject, or in accordance with the state of the respiratory organs of the measurement subject.

Further, the correction value can be set empirically on the basis of clinical data or analytical data or the like that is obtained from a group of subjects of measurement having individual information and similar information such as nationality, sex, age, height, weight, and current state of the respiratory organs, of the subjects of measurement. The correction value can be set to be 2% for example. However, the present disclosure is not limited to this in actuality, and the correction value can be changed in accordance with the measurement subject, or in accordance with the state of the respiratory organs of the measurement subject.

In a case in which, as described above, the measurement subject is a patient who has contracted a specific viral infection, and the set initial stage value is 92% for example and the set correction value is 2%, a value of "90%" can be obtained by subtracting the correction value from the initial stage value. The obtained value of "90%" can be set as the reference value for judging whether or not a warning is to be given in the oxygen saturation measurement.

Further, the congruity between the accuracy of measuring at the position of the wrist and the accuracy of measuring at the position of a fingertip was evaluated by Bland-Altman analysis using five subjects of measurement as subjects. Specifically, in a state in which each measurement subject was holding their breath, the changes over time in the measured value of the oxygen saturation at the position of the wrist, and the changes over time in the measured value of the oxygen saturation at the position of a fingertip, were acquired by using the first sensor portion 11.

In the Bland-Altman analysis, the average value of the values of the oxygen saturations of the five subjects of measurement was computed, and the extent of the plus/minus error from the computed average value was computed within the range of 80% or more and 100% or less. The extent of the error in the case of measuring at the position of a fingertip was around 3.0%.

On the other hand, the extent of the error in the case of measuring at the position of the wrist was around 6.0%. Namely, it was understood that, in the case of measuring at the position of the wrist, the extent of the error was around two times greater than that in the case of a fingertip. Further, from the results of analysis, it was understood that the error of the measurement at the position of the wrist was even greater than the error of measurement at the position of a fingertip particularly within the range of an oxygen saturation of 90% or less.

As illustrated in Fig. 5, it can be understood that, in a case in which the oxygen saturation that is the reference value is 90% or less, the accuracy of measuring the oxygen saturation at the time of measuring at the position of the wrist is lower than the accuracy of measurement at the time of measuring at the position of a fingertip. The perfusion of arterial blood at the position of a fingertip is greater than the perfusion of arterial blood at the position of the wrist. Therefore, the accuracy of a judgement on the state of the respiratory organs of a measurement subject deteriorates. On the other hand, in a case in which the oxygen saturation that is the reference value for judging whether or not a warning is to be given exceeds 94%, the range over which the state of the respiratory organs is diagnosed to be normal increases, and therefore, the number of times of warnings increases.

In the present disclosure, the condition used in judging whether or not a warning is to be given is not limited to a minimum value of the oxygen saturation and can be set arbitrarily. For example, the condition may be set using biometric information other than the oxygen saturation, such as the respiration rate or the number of times of coughing. For example, the respiration rate and the number of times of coughing of a measurement subject are biometric information that can be acquired from pulse wave signals.

Further, the condition may be set by using arbitrary blood vessel information other than pulse wave signals, such as, for example, sound wave information of the sound of an artery which is the coursing through a blood vessel. For example, in a case of using the coursing sound of an artery, a microphone having a sound collecting function may be provided at the first sensor portion 11. For example, the frequency or amplitude can be used as the sound wave information. Further, it is also possible to acquire the number of times of coughing from the coursing sound of an artery. The reference value that is used in condition setting can be set on the basis of sound wave information from the coursing sound of an artery.

In the present embodiment, the warning may be image information including words or graphics or the like. Note that, in the present disclosure, the warning is not limited to image information, and may be carried out by light that is lit or flashes a specific warning color. Further, the warning may be a sound or a vibration.

(Second Sensor Portion)

As illustrated in Fig. 1 and Fig. 2, the second sensor portion 21 is attached to the outer side of the band 12, i.e., the side of the band 12 that does not contact the wrist, at a position that can be visually confirmed easily by the measurement subject. The second sensor portion 21 measures the oxygen saturation of the artery of a fingertip of the measurement subject. The second sensor portion 21 of the present embodiment is structured by a measuring portion 28 and the computing section 18. The measuring portion 28 has a panel serving as a touchscreen interface. Note that, in the present disclosure, the mounted position of the second sensor portion is not limited to the outer side of the band 12. The second sensor portion can be mounted even to the inner side of the band 12 provided that erroneous operation of the touchscreen due to, for example, a non-energized state of the second sensor portion arising while the second sensor portion is contacting the wrist, is prevented.

Unillustrated light energy sources are provided at the measuring portion 28, and lights of two types of wavelengths for oxygen saturation measurement are irradiated from the light energy sources at the interior of the measuring portion 28. Attenuation occurs respectively at the lights of the two types of wavelengths, due to a fingertip of the measurement subject contacting the measuring portion 28. Further, an unillustrated sensing portion that senses the attenuated lights is provided at the measuring portion 28, and the sensing portion is connected to the computing section 18. On the basis of the attenuated lights, the computing section 18 measures the oxygen saturation within the arterial blood of the fingertip.

Note that, in the present disclosure, the second sensor portion can be structured arbitrarily provided that it can measure the oxygen saturation of an artery of a fingertip. For example, the method of measuring oxygen saturation at the second sensor portion may be a type that reflects or a type that transmits the measurement light. Further, a known touchscreen interface technique may be applied to the second sensor portion.

<Oxygen Saturation Measuring Method>

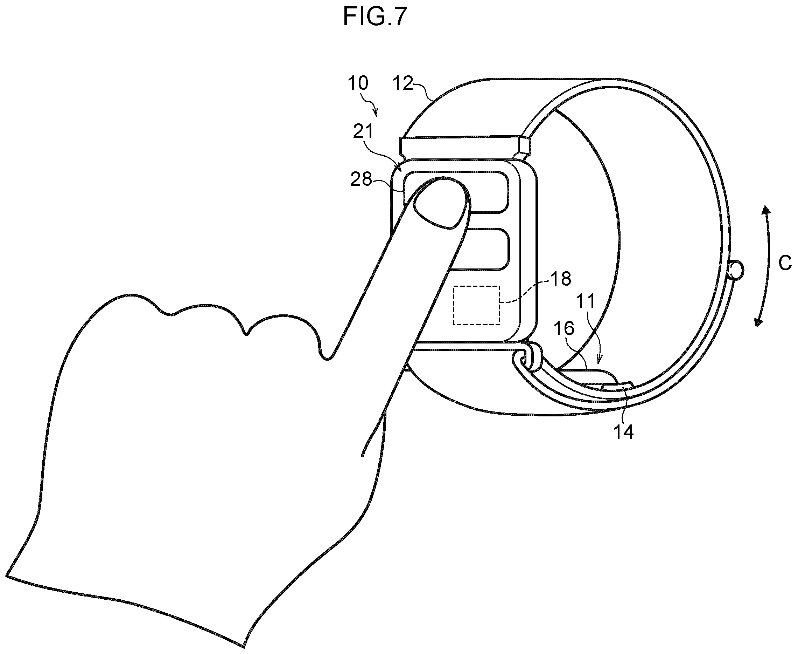

An example of an oxygen saturation measuring method using the oxygen saturation measuring device 10 relating to the present embodiment is described with reference to Fig. 2 and Fig. 6 through Fig. 7.

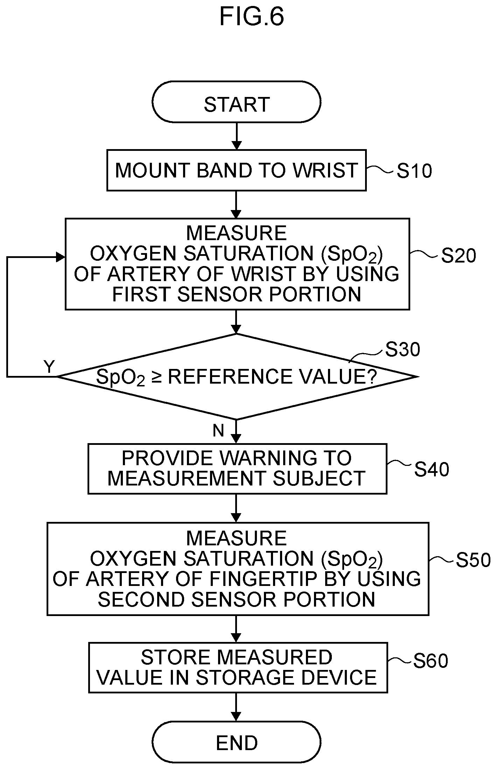

First, as shown in step S10 of Fig. 6, the measurement subject winds the band 12 of the oxygen saturation measuring device 10 on a wrist of the measurement subject. The artery that is the target of measurement is the radial artery 24 for example. Note that, in the present disclosure, the artery that is the target of measurement is not limited to the radial artery 24, and may be another, arbitrary artery. Further, the contacting surfaces 16A are made to contact the skin in a vicinity of the radial styloid process 20A at the inner side of the wrist.

Next, as illustrated in Fig. 2, the measurement subject places the contacting portions 16 at the inner side of the hollow of the gap between the radial styloid process 20A and the flexor carpi radialis tendon 22. Next, as shown in step S20, by using the first sensor portion 11, the measurement subject acquires the pulse wave signals of an artery of the wrist of the measurement subject. Then, the oxygen saturation of the artery is measured on the basis of the acquired pulse wave signals.

Specifically, light is irradiated from the light-emitting elements toward the radial artery 24 that is positioned at the lower side of the hollow in Fig. 2. Then, the light reflected from the radial artery 24 is received by the light-receiving elements. Then, the computing section 18 measures the oxygen saturation on the basis of the pulse wave signals acquired from the reflected light.

Next, in step S30, the computing section 18 compares the measured oxygen saturation with a preset reference value. If the oxygen saturation that is compared is the reference value or greater, it is judged that a warning is not necessary. Then, the processing moves on to step S20, and measuring of the oxygen saturation is repeated. On the other hand, if the oxygen saturation that is compared has not reached the reference value, as shown in step S40, the computing section 18 issues a warning to the measurement subject.

Here, in the present disclosure, for example, the energizing of the second sensor portion 21 may be controlled such that, up until a time that a warning is given, energization of the second sensor portion 21 is blocked, and after a warning is given, energization of the second sensor portion 21 is started. The amount of electric power that is consumed can be kept in check by controlling the energizing of the second sensor portion 21.

Next, in shown in step S50, the measurement subject measures the oxygen saturation of an artery of a fingertip by using the second sensor portion 21. Specifically, the measurement subject makes a fingertip contact the measuring portion 28 of the second sensor portion 21. The measured value of the measured oxygen saturation is displayed on the notification portion 26 that serves as a display portion. Note that, in the present disclosure, the display portion that displays the measured value of the oxygen saturation measured by the second sensor portion 21 may be integral with the notification portion 26 such as in the present embodiment, or may be provided so as to be different from the notification portion 26.

Next, as shown in step S60 of Fig. 6, the computing section 18 stores the measured value in a storage device. Having the notification portion 26 display stored, measured values afterwards at the time of an examination by a doctor or the like for example, can contribute to the examination.

Note that, in the present disclosure, arbitrary, additional data, such as for example the name of the sensor portion used in measurement or the date and time, may be stored in the storage device together with the measured value. Further, in the present disclosure, the measured value that is measured by the first sensor portion 11 may be stored in the storage device. Further, in the present disclosure, for example, the processings of step S20 and steps thereafter may be repeated after the processing of step S60.

(Operation/Effects)

In accordance with the first aspect, the band 12 is wound around the wrist of a measurement subject, and the pulse wave signals of an artery of the wrist are acquired by the first sensor portion 11 that is attached to the band 12. The oxygen saturation of the artery of the wrist is measured on the basis of the acquired pulse wave signals. Namely, because the oxygen saturation is measured at the position of the wrist, as compared with a case of measuring the oxygen saturation at the position of a fingertip, it is difficult for a state in which actions of work or actions of daily life of the measurement subject are impeded to arise, and it is easy to continuously measure the oxygen saturation over time.

Further, the measured oxygen saturation of the artery of the wrist is compared with a preset reference value, and, in a case in which the measured oxygen saturation has not reached the reference value, a warning expressing the possibility of the respiratory organ disorder is given to the measurement subject. Due to the warning, the measurement subject is urged to take action such as conferring with a doctor for example. Therefore, as a result, an improvement in the state of the respiratory organs can be devised.

Further, in the present embodiment, the first sensor portion 11 is attached to the inner side of the band 12, i.e., to the side of the band 12 that contacts the wrist. Therefore, it is easy to measure the radial artery 24 that is near to the surface at the inner side of the wrist.

Further, in the present embodiment, in a case in which the oxygen saturation is less than 90%, urging of the measurement subject to measure the oxygen saturation at the position of a fingertip is given to the measurement subject as a warning. Therefore, measuring the oxygen saturation with a higher measurement accuracy can be promoted.

Further, the oxygen saturation measuring device 10 relating to the present embodiment further has the second sensor portion 21 that is attached to the band 12 and measures the oxygen saturation of an artery of a fingertip. Therefore, both the continuous measuring over time of the oxygen saturation at the position of the wrist, and the measuring of the oxygen saturation at the position of a fingertip with higher measuring accuracy, can be realized by the one oxygen saturation measuring device 10. Namely, there is no need to separately ready an oxygen saturation measuring device for measuring the oxygen saturation of an artery of a fingertip.

Further, even in a case in which a highly accurate oxygen saturation measuring device such as a device that measures at the position of a fingertip is disposed at a place that is apart from the measurement subject, there is no need for the measurement subject to move in order to retrieve the highly-accurate oxygen saturation measuring device. Therefore, after a warning is given, the measurement subject can rapidly start measuring the oxygen saturation of an artery of their fingertip. Further, because the bother of the measurement subject moving in order to retrieve an oxygen saturation measuring device can be eliminated, it is easy for the measurement subject to establish the habit of measuring their oxygen saturation.

Further, in the present embodiment, the second sensor portion 21 is attached to the outer side of the band 12, i.e. the side of the band 12 that does not contact the wrist, at a position that can be visually confirmed easily by the measurement subject. Therefore, as compared with a case in which the second sensor portion 21 is attached to the inner side of the band 12, handling of the second sensor portion 21 by the measurement subject is easy.

Further, in the present embodiment, the notification portion 26 and the display portion form an integrated body. Therefore, the overall dimensions of the oxygen saturation measuring device are reduced as compared with a case in which the notification portion 26 and the display portion are separate bodies. As a result the oxygen saturation measuring device can be structured to be compact.

Further, in accordance with an oxygen saturation measuring method that uses the oxygen saturation measuring device 10 relating to the present embodiment, it is easy to continuously measure the oxygen saturation over time, and an improvement in the state of respiratory organs can be devised.

(Modified Example)

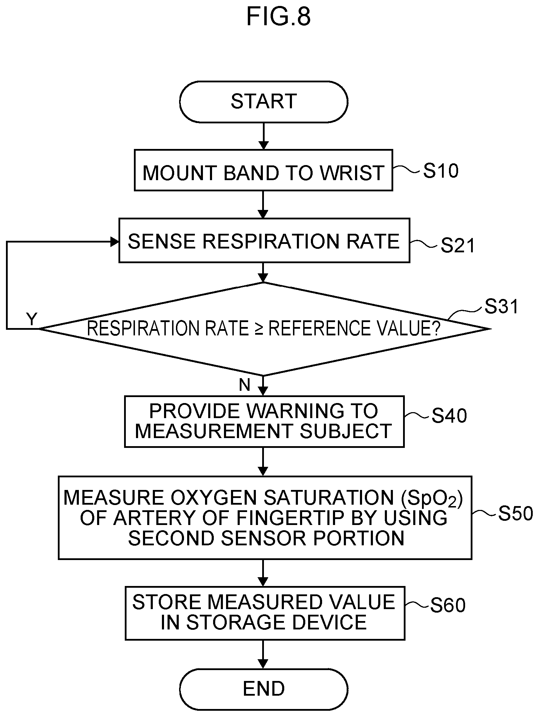

The present embodiment describes, as an example, a case in which the condition for providing a warning to measure the oxygen saturation at the position of a fingertip is that the oxygen saturation measured at an artery of the wrist of the measurement subject has not reached a reference value. However, the present disclosure is not limited to this. For example, Fig. 8 exemplifies an oxygen saturation measuring method relating to a modified example in which a warning is given on the basis of the state of respiration of the measurement subject.

The oxygen saturation measuring method relating to the modified example differs from the oxygen saturation measuring method relating to the present embodiment mainly with regard to the point that the method relating to the modified example includes step S21 and step S31 of Fig. 8 instead of step S20 and step S30 of Fig. 6. In step S21 and step S31, the respiration rate is used instead of the oxygen saturation. Because the contents of steps other than step S21 and step S31 are similar to the respectively corresponding steps of the same names in Fig. 6, repeat description is omitted.

In the modified example, as shown in step S21 of Fig. 8, the respiration rate of the measurement subject is sensed. For example, the pulse wave signals of an artery of the wrist of the measurement subject are acquired by using the first sensor portion 11, and sensing of the respiration rate can be carried out on the basis of the acquired pulse wave signals.

Next, in shown in step S31, the sensed respiration rate and the respiration rate that is a preset reference value are compared. In a case in which the respiration rate that is compared is the reference value or greater, it is judged that a warning is unnecessary. In a case in which it is judged that a warning is unnecessary, the processing moves on to step S21, and measuring of the respiration rate is repeated.

On the other hand, in a case in which the respiration rate that is compared has not reached the reference value, a warning is given to the measurement subject as shown in step S40. Thereafter, processings that are similar to those of the present embodiment are carried out. In the modified example, even in a state in which the oxygen saturation cannot be measured, the state of the respiratory organs of the measurement subject can be judged by using the respiration rate. In the present disclosure, the condition can be set in advance by using biometric information other than the oxygen saturation as in the modified example.

<Other Embodiments>

Although the present disclosure has been described by the above-disclosed embodiment, the description and drawings that form a part of the disclosure are not to be construed as limiting the present disclosure. For example, in the present disclosure, the means for acquiring pulse wave signals of an artery of the wrist is not limited to a wristwatch-type device using a band. For example, the oxygen saturation measuring device may be a portable device that can be set close to the wrist. Further, the means for acquiring pulse wave signals of an artery of the wrist does not have to be device that is used exclusively for measuring oxygen saturation, and, for example, may be a general-purpose device that has an oxygen saturation measuring function. Namely, the method of measuring oxygen saturation is not limited to a device.

Further, in a case in which a warning is given on the basis of the state of respiration of the measurement subject, a necklace-type respiration rate measuring device that can be set close to the chest portion of the measurement subject or the like for example also can be employed. Further, the notification portion is not essential to the present disclosure. For example, the oxygen saturation measured by the second sensor portion may be transmitted to the exterior by a communicating function, and the transmitted oxygen saturation may be displayed on a display device that serves as a notification portion and is provided at the exterior. Further, the present disclosure may be structured even if the second sensor portion is not used.

The present disclosure includes various embodied forms and the like that have not been described above, and the technical scope of the present disclosure is determined only by the pertinent matters specifying the invention of the Claims from the above description.

The disclosure of Japanese Patent Application No. 2022-115117 filed on July 19, 2022 is, in its entirety, incorporated by reference into the present specification.

All publications, patent applications, and technical standards mentioned in the present specification are incorporated by reference into the present specification to the same extent as if such individual publication, patent application, or technical standard was specifically and individually indicated to be incorporated by reference.

**Claims**

1. An oxygen saturation measuring device comprising:

   a band wound on a wrist of a measurement subject;
   a first sensor portion attached to the band, the first sensor portion acquiring pulse wave signals of an artery of the wrist, and measuring oxygen saturation of the artery of the wrist on the basis of the acquired pulse wave signals; and
   a notification portion that, in a case in which the oxygen saturation measured by the first sensor portion satisfies a preset condition, provides the measurement subject with a warning expressing a possibility of respiratory organ disorder.

2. The oxygen saturation measuring device of Claim 1, wherein the first sensor portion is attached to an inner side of the band.

3. The oxygen saturation measuring device of Claim 1 or 2, wherein, in a case in which the measured oxygen saturation does not reach a preset reference value, the notification portion provides, as the warning, a notice urging the measurement subject to measure oxygen saturation at a position of a fingertip.

4. The oxygen saturation measuring device of Claim 1 or 2, further comprising a second sensor portion attached to the band and measuring oxygen saturation of an artery of a fingertip of the measurement subject.

5. The oxygen saturation measuring device of Claim 4, wherein the second sensor portion is attached to an outer side of the band.

6. An oxygen saturation measuring device comprising:

a band wound on a wrist of a measurement subject;
a first sensor portion attached to the band, the first sensor portion acquiring pulse wave signals of an artery of the wrist, and measuring oxygen saturation of the artery of the wrist on the basis of the acquired pulse wave signals;
a notification portion that, in a case in which the oxygen saturation measured by the first sensor portion satisfies a preset condition, provides the measurement subject with a warning expressing a possibility of respiratory organ disorder;
a second sensor portion attached to the band and measuring oxygen saturation of an artery of a fingertip of the measurement subject; and
a display portion displaying the oxygen saturation measured by the second sensor portion.

7. The oxygen saturation measuring device of Claim 6, wherein the notification portion and the display portion form an integrated body.

8. An oxygen saturation measuring method comprising:

acquiring pulse wave signals of an artery of a wrist of a measurement subject;
measuring oxygen saturation of the artery on the basis of the acquired pulse wave signals; and
in a case in which the measured oxygen saturation satisfies a preset condition, providing the measurement subject with a warning.

# FIG.1

FIG.2

# FIG.3

FIG.4

FIG.5

# FIG.6

```
              ┌─────────────┐
              │    START    │
              └──────┬──────┘
                     │
          ┌──────────▼──────────┐
          │ MOUNT BAND TO WRIST │ ──S10
          └──────────┬──────────┘
                     │
      ┌──────────────▼──────────────┐
      │          MEASURE            │
      │ OXYGEN SATURATION (SpO₂)    │ ──S20
      │ OF ARTERY OF WRIST BY USING │
      │     FIRST SENSOR PORTION    │
      └──────────────┬──────────────┘
                     │
  Y           ┌──────▼──────┐  S30
  ◄───────────┤ SpO₂ ≥ REFERENCE VALUE? ├
              └──────┬──────┘
                     │ N
      ┌──────────────▼──────────────┐
      │     PROVIDE WARNING TO      │ ──S40
      │    MEASUREMENT SUBJECT      │
      └──────────────┬──────────────┘
                     │
      ┌──────────────▼────────────────┐
      │           MEASURE             │
      │  OXYGEN SATURATION (SpO₂)     │ ──S50
      │ OF ARTERY OF FINGERTIP BY USING│
      │    SECOND SENSOR PORTION      │
      └──────────────┬────────────────┘
                     │
      ┌──────────────▼──────────────┐
      │     STORE MEASURED          │ ──S60
      │ VALUE IN STORAGE DEVICE     │
      └──────────────┬──────────────┘
                     │
              ┌──────▼──────┐
              │     END     │
              └─────────────┘
```

FIG.7

EP 4 559 392 A1

# FIG.8

```
                    ┌──────────────┐
                    │    START     │
                    └──────┬───────┘
                           │
                           ▼
              ┌───────────────────────────┐
              │   MOUNT BAND TO WRIST     │──S10
              └───────────┬───────────────┘
                          │
                          ▼
         ┌─────────────────────────────────┐
    ┌───▶│   SENSE RESPIRATION RATE        │──S21
    │    └───────────┬─────────────────────┘
    │                │
    │                ▼
    │ Y        ╱◇◇◇◇◇◇◇◇◇◇◇◇◇◇◇◇◇◇◇◇◇◇◇◇╲         S31
    └────────◇  RESPIRATION RATE ≥ REFERENCE VALUE?  ◇
              ╲◇◇◇◇◇◇◇◇◇◇◇◇◇◇◇◇◇◇◇◇◇◇◇◇╱
                          │ N
                          ▼
              ┌───────────────────────────┐
              │   PROVIDE WARNING TO      │──S40
              │   MEASUREMENT SUBJECT     │
              └───────────┬───────────────┘
                          │
                          ▼
         ┌─────────────────────────────────────┐
         │ MEASURE OXYGEN SATURATION (SpO₂)    │──S50
         │ OF ARTERY OF FINGERTIP BY USING     │
         │ SECOND SENSOR PORTION               │
         └───────────┬─────────────────────────┘
                     │
                     ▼
              ┌───────────────────────────┐
              │ STORE MEASURED VALUE      │──S60
              │ IN STORAGE DEVICE         │
              └───────────┬───────────────┘
                          │
                          ▼
                    ┌──────────────┐
                    │     END      │
                    └──────────────┘
```

**EP 4 559 392 A1**

## INTERNATIONAL SEARCH REPORT

| International application No. |
|---|
| **PCT/JP2023/025792** |

| A. | CLASSIFICATION OF SUBJECT MATTER |
|---|---|

***A61B 5/1455***(2006.01)i; ***A61B 5/02***(2006.01)i; ***A61B 5/08***(2006.01)i
FI: A61B5/1455; A61B5/02 310A; A61B5/08

According to International Patent Classification (IPC) or to both national classification and IPC

| B. | FIELDS SEARCHED |
|---|---|

Minimum documentation searched (classification system followed by classification symbols)

A61B5/145-5/1455; A61B5/00-5/03; A61B5/08

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2017-153879 A (SEIKO EPSON CORP.) 07 September 2017 (2017-09-07) paragraphs [0001], [0014]-[0017], [0020], [0021], [0027], [0029]-[0031], [0036], fig. 1, 2, 7 | 1-2, 8 |
| A | paragraphs [0001], [0014]-[0068], fig. 1-16 | 3-7 |
| A | JP 2015-107152 A (PACIFIC MEDICO CO., LTD.) 11 June 2015 (2015-06-11) paragraphs [0014]-[0060], fig. 1-4 | 1-8 |
| A | JP 2007-319247 A (OMRON HEALTHCARE CO., LTD.) 13 December 2007 (2007-12-13) paragraphs [0032]-[0174], fig. 1-34 | 1-8 |

| ☐ Further documents are listed in the continuation of Box C. | ☑ See patent family annex. |
|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **07 September 2023** | **19 September 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

21

**INTERNATIONAL SEARCH REPORT**
Information on patent family members

International application No.

**PCT/JP2023/025792**

| Patent document cited in search report | | | Publication date (day/month/year) | Patent family member(s) | Publication date (day/month/year) |
|---|---|---|---|---|---|
| JP | 2017-153879 | A | 07 September 2017 | US 2017/0251928 A1 paragraphs [0002], [0032]-[0035], [0038], [0039], [0045], [0047]-[0049], [0054], fig. 1, 2, 7 | |
| JP | 2015-107152 | A | 11 June 2015 | (Family: none) | |
| JP | 2007-319247 | A | 13 December 2007 | WO 2017/138799 A1 paragraphs [0012]-[0154], fig. 1-34 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010246894 A
- JP A A

- JP 2022115117 A